# EUROPEAN PATENT APPLICATION

(11) **EP 2 662 441 A1**
(43) Date of publication of application: **13.11.2013**
(21) Application number: 12167799.1
(22) Date of filing: 12.05.2012
(51) Int. Cl.: C12N 5/0784, A61K 35/14, A61K 39/39, A61K 39/108, A61K 38/21, A61K 31/713, A61K 31/38

(54) **Method for the in vitro maturation of dendritic cells**

(71) Applicant: Universiteit Maastricht, 6211 LK Maastricht (NL); Academisch Ziekenhuis Maastricht, 6229 HX Maastricht (NL)
(72) Inventor: Bos, Gerardus Marinus Josef, 6229 ER Maastricht (NL); Germeraad, Wilfred Thomas Vincent, 6229 ER Maastricht (NL); Gijsbers, Lamberta Maria Gerardina, 6229 ER Maastricht (NL); Oth, Tammy, 6229 ER Maastricht (NL); Vanderlocht, Joris, 6229 ER Maastricht (NL)
(74) Representative: Habets, Winand

(57) **Abstract**

The invention is in the field of cell biology and immunology. The invention provides methods for the production of a vaccine for cancer and infectious diseases. More in particular it provides a method for the production of mature dendritic cells wherein immature dendritic cells are exposed to a composition comprising FMKp and interferon gamma in combination with CL075 and/or Poly IC in a culture medium for a sufficient amount of time and wherein mature dendritic cells are isolated from the culture medium.

## Description

### Field of the invention

The invention is in the field of cell biology and immunology. The invention provides methods for the production of a vaccine for cancer and infectious diseases. More in particular it provides a method for the improved production of dendritic cells.

### Background of the invention

Despite improvements in conventional cancer treatments, such as surgery, chemo- and radiotherapy, cancer is still a leading cause of death worldwide (1). Therefore, there is an urgent need for more effective and less invasive therapies. A growing field of interest in the search for novel therapeutic strategies is that of immunotherapy. As the immune system of many cancer patients is suppressed, the first line of defense against malignant cells is lost. This creates a tumor permissive environment.

Immunotherapy aims to overcome this suppression and reactivate the patient's own immune system to reject the tumor. There are various strategies already being used in the clinic, utilizing both active and passive immunity.

The treatment of cancer with monoclonal antibodies is one example of passive immunotherapy. Monoclonal antibodies to known tumor antigens are injected, where they recognize and bind to their specific epitope. These antibodies are used alone, relying on antibody-dependent cell-mediated cytotoxicity or an anti-apoptotic signal, or are coupled to therapeutic agents allowing for localized administration. Monoclonal antibodies have shown positive results on both long-term and short-term survival, although treatment often needs to be coupled to radiotherapy. The downside of this strategy is that most tumor-associated epitopes are located within the cell, making them inaccessible for antibodies. Additionally, few antigens are tumor-specific resulting in unwanted targeting of healthy cells.

Other examples of passive immunotherapies are immune adjuvants and cytokines. These therapies aim at creating an inflammatory environment that would counteract the immunosuppressive nature of the malignancy, allowing spontaneous immune responses to take place. However, results using these therapies are limited. The downside of passive immunotherapies in general, is that memory immune cells are not formed, making relapse likely.

These disadvantages may be circumvented by active immunotherapy, which initiates an immune response against the tumor, thereby not only making use of the (antigen-specific) effector cells, such as cytotoxic lymphocytes (CTL), plasma cells and NK cells, but also of protective memory. One strategy for active immunotherapy is through vaccination with dendritic cells (DC). These cells are the most important antigen-presenting cells in the body, capable of initiating both innate and adaptive immune responses.

In cancer patients, DC have been shown to be impaired in their activity, making them attractive targets for immunotherapy (2). These and other immunotherapies, are extensively reviewed in literature references 3 and 4.

DC-based vaccines are already in use in the clinic, revealing no or only mild side-effects (5). However, only 10 - 15% of the patients thus treated responded to the treatment (5). This indicates that improvements in DC-based vaccination protocols remain of vital importance if large scale tumor eradication is to be achieved.

Dendritic cells are a heterogeneous group of cells in the body that differ in localization, phenotype and function. In the peripheral blood, the two main types are the CD11c- plasmacytoid DC (pDC) and CD11 c+ myeloid DC (myDC). Although pDC comprise only 0.1% of all peripheral blood mononuclear cells (PBMC), they are crucial in antiviral immunity through the production of large quantities of type I interferons (6; 7). myDC can be further subdivided into cells that differentially express BDCA1 (CD1c), CD16, and BDCA3 (8; 9). Although CD16+-myDC comprise 65-75% of the total myDC population, BDCA1+-myDC (10-20%) are best described in literature and are thought to be the most potent T cell stimulators of the three myDC subpopulations (8).

In vivo, monocytes are capable of differentiating into myDC after sensing an inflammatory signal, maintaining a stable number of DC in the blood (7; 10). Ex vivo, monocyte-derived immature DC (iDC) are obtained by culturing monocytes in the presence of different growth factors (10).

Depending on the factors added, monocytes differentiate into DC with different functions and phenotypes (reviewed in (10)). Due to the low number of circulating DC in the blood, most DC-based vaccine studies use these ex vivo cultured iDC. The current standard cocktail for inducing monocyte activation and differentiation towards iDC contains GM-CSF and IL-4.

As professional antigen presenting cells (APC), DC have a central role in activating the innate and adaptive immune system and are able to tailor the immune response to the specific nature of a pathogen. iDC take up pathogens via endocytosis, process it, and present small peptide fragments (antigens) of that specific pathogen on their cell surface through MHC class I and class II molecules.

Upon pathogen recognition, DC undergo maturation. This process involves downregulating their phagocytic ability, while upregulating production of inflammatory cytokines and expression of surface molecules, such as co-stimulatory molecules and chemokine receptors (11). The nature of the pathogen dictates which cytokines and surface molecules are expressed by the DC. This process is mediated by pattern recognition receptors (PRR; see section 1.5) and allows the DC to initiate an immune response tailored to the specific pathogen. The migration of mature DC (mDC) towards the lymph nodes contributes to their ability to mount immune responses, because the architecture of these organs is such that encounters of mDC with adaptive immune effector cells are maximized (12).

In tumor immunity, it is important that DC mount a potent T_{H}1-polarized response to maximize the antigen-specific tumor killing by CTL (13). More recently, the importance of recruiting and activating NK cells has come to light, as they are not only capable of lysing tumor cells, but also help propagate the TH1-response.

NK cells are part of the innate immune system and play an important role in the immune surveillance of transformed cells, as they provide a first line of defense for virally transformed and malignant cells (14). The activation of NK cells is regulated by a delicate balance between activating and inhibiting receptors. In healthy cells, the balance favors inhibition, thereby protecting the cell from lysis (15). The activation of NK cells occurs either by the loss of inhibitory signals or by the gain of activating signals, providing two distinct mechanisms through which NK cells can perform their function (15). Inhibitive signals are provided to NK cells by the recognition of MHC class I molecules by Killer-cell immunoglobulin-like receptors (KIRs), and of non-classical HLA-E by NKG2A (16).

Each KIR recognizes a specific HLA-molecule and NK cells are known to be heterogeneous in their KIR expression (16). This specificity and distribution allows for continual survey of each HLA-molecule by NK cells. Both virally infected and malignant cells are known to downregulate MHC class I molecules to escape detection by the adaptive immune system (17). This downregulation results in the loss of inhibitory signals in NK cells, which become activated and lyse the cell. The activation of NK cells through loss of inhibition is referred to as "missing-self-recognition". Receptors that are involved in relaying activating signals include natural cytotoxicity receptors (e.g. NKp30, NKp40 and NKp46), NKG2D, and 2B4. Functional studies indicate that malignancies often express ligands to these receptors (16). The presence of these ligands could stimulate the receptors to a degree that the balance will tip towards activation and overrule and inhibitory signals provided by MHC-molecules, thereby activating NK cells. This pathway of NK cell activation is known as "induced-self-recognition".

In addition to their immune surveillant function for transformed cells, NK cells also have an immunoregulatory function by producing cytokines, such as TNF-alpha and IFN-gamma. This function of NK cells is especially important in NK-DC crosstalk. This crosstalk results in the reciprocal enhancement of functions, and has received increasing interest as an important factor in anti-tumor immunity. On the one hand DC activate NK cells by secreting cytokines, such as IL-12, IL-18, and IL-15 (with IL-2). IL-15 was shown to enhance NK cell survival (18), while IL-12 promoted NK cell IFN-gamma production (19; 20). Additionally, DC can recruit NK cells to the secondary lymph nodes in a CCR5 dependent manner (21), where NK cells provide an early source of IFN-gamma that aids TH1-polarization and CTL priming by DC (22; 23). These effects of NK-DC crosstalk on T cells are crucial in anti-tumor immunity, as they prime TH1 cells and CTL to eradicate the tumor in an antigen-specific manner (24). Subsequently, trials that using DC that activate NK cells show an enhanced anti-tumor activity (25; 26).

On the other hand, NK cells are able to kill immunosuppressive iDC, as well as inappropriately matured DC, thereby ensuring the quality of the immune response (27). Under certain circumstances, NK cells are also capable of maturing DC by the secretion of TNF-alpha and IFN-gamma (20). These DC express high levels of CCR7, thereby facilitating migration to the secondary lymph nodes (20). Through these different interactions with DC, NK cells are capable of modulating the adaptive immune response. NK-DC crosstalk is mainly thought to take place in abused tissue and secondary lymph nodes, with responses differing according to the site of encounter (28).

DC and other cells of the innate immune system, such as NK cells, need to be able to directly identify specific pathogens in order to activate the appropriate immune response (31). They are capable of doing this via pattern recognition receptors (PRR), which recognize highly conserved pathogen associated molecular patterns (PAMP), typical for pathogens and that are never present in a healthy host. Depending on the PAMP encountered, different PRR and their signaling pathways become activated.

Cells differ in their expression of PRR, making them differentially susceptible to pathogens. Due to their central role in the immune system, DC express a wide array of PRR, including Toll-like receptors (TLR), nucleotide-binding oligomerization domain (NOD)-like receptors, C-type lectin receptors (CLR) and retinoic acid-inducible gene 1 (RIG-1)-like receptors. These receptors recognize different classes of pathogens, which ensures the induction of the appropriate immune response (30).

The best described PRR are members of the Toll-like receptor family, which can be found both intra- and extracellularly. The intracellular TLR 3, TLR 7, TLR 8, and TLR 9 are located in endosomes and recognize bacterial and viral DNA or RNA. The extracellular TLR 1, TLR 2, TLR 4, TLR 5 and TLR 6 are located on the cell surface and recognize membrane components of pathogens, such as LPS, peptidoglycan, and flagellin. All the TLR, except TLR3, signal in a Myd88-dependent manner, leading to NF-kappa B activation. In addition, TLR3 and TLR4 activate NF-kappa B and other transcription factors important for type IIFN production, via TRIF (31).

CLR can be found on the cell-membrane and are part of the fungal recognizing mechanism of the immune system, ultimately activating NF-kappa B. They also play an important role in antigen-uptake and presentation. More recently they have been shown to be important regulators of DC migration (32).

NOD- and RIG-like receptors are found in the cytoplasm, recognizing viral and bacterial components that are present in the cell. Their activation triggers the production of type I IFN through NF-kappa B (33; 34).

Depending on the PAMP encountered, different PRR and their signaling pathways become activated. One pathogen typically activates more than one PRR. Depending on the PRR combination triggered, a distinct signaling cascade is initiated that dictates the immune response and maturation program of the DC. This maturation program regulates the expression of co-stimulatory molecules, pro-inflammatory cytokines, chemokines and their receptors (30). The fact that different DC subsets vary in their adequacy to respond to different triggers can be explained by the fact that they are not equal in their expression of PRR (reviewed in 7). It is thought that triggering specific combinations of PRR will lead to the maturation of DC most suitable for anti-cancer vaccines, leading to potent TH1-skewing and NK cell responses. However, it is unknown which combination of PRR is optimal for inducing this effect.

Research has focused on identifying PRR-triggers that are capable of producing a mature DC phenotype with a predominant IL-12 cytokine profile that can enhance CTL activation and NK cell responses by providing a TH1-response. It has been established that the addition of membrane fragments of *Klebsiella pneumoniae* (FMKp; TLR2/4-trigger) together with IFN-gamma to the DC maturation cocktail, produces DC that are superior to the "golden standard" which are DC matured in the presence of a TNFalpha/PGE2-cocktail. The FMKp/IFN-gamma cocktail has proven to be in vitro superior in regard to CTL activation (35), IL-12 secretion and subsequent TH1-polarization (36). FMKp/IFN-gamma-matured DC were shown to produce more chemokines capable of attracting other immune cells (35).

One of the challenges that face DC-based vaccines is the immunosuppressive microenvironment of the tumor (17). DC have to overcome this suppressive activity in order to activate immune effector cells.

Many malignancies actively secrete immunosuppressive factors, amongst which prostaglandin E2 (PGE2) (36). This creates a permissive state in which the tumor can grow, whilst escaping detection by the immune system. PGE2 has been shown to modulate the adaptive immune system by favoring the propagation of TH2/ TH17 above a TH1-response (37; 38). Additionally, it decreases the cytotoxic capacity of CTL (39), as well as inhibiting maturation and antigen-presentation of DC (40). Besides the effect on T cells, it also has a negative effect on NK cell function by directly binding to the EP receptors on the NK cell membrane (41). This has been shown to reduce NK cell proliferation, cytotoxicity and migration, as well as inhibiting IFN-gamma secretion (42). Although these negative effects on NK cells are described, it is yet unclear how NK-DC crosstalk is influenced by PGE2.

Paradoxically, the "golden standard" cocktail for maturing DC ex vivo in current clinical trials contains PGE2. This cocktail produces a fully mature DC phenotype, with the ability to in vitro migrate towards a CCL-19/21 chemokine gradient, indicating that they could be effective at migrating towards the lymph nodes (35). However, clinical results show that this migratory ability is limited (43). In addition, these DC are poor producers of IL-12, necessary for TH1-polarization (44).

It may therefore be concluded that there is a great need for maturation cocktails that are better able to produce mature DC, in particular DC capable of attacking a tumor.

### Summary of the invention

We found that a certain combination of maturation agents in a cocktail synergistically improves the production and maturation of dendritic cells. This may be used in the production of dendritic cell-based vaccines for the treatment of cancer and infectious diseases.

Accordingly, the invention relates to an ex vivo method for the production of mature dendritic cells wherein immature dendritic cells are exposed to a composition comprising FMKp and interferon gamma in combination with CL075 and/or Poly IC in a culture medium for a sufficient amount of time and wherein mature dendritic cells are isolated from the culture medium.

The maturation cocktail as described herein may also be used as a pharmaceutically active preparation in the treatment of humans or animals. Hence, the invention also relates to a composition comprising FMKp and interferon gamma in combination with CL075 and/or Poly IC for use in the induction of an immune response in an animal or human.

The invention may also be used to obtain superior dendritic cells. Hence, the invention also relates to mature dendritic cells obtainable by the method according to the invention.

### Legends to the figures

Figure 1: Graph showing the dose-dependent influence of addition of IL12 to the IFN gamma production of Th 1 cells.
Figure 2: graph showing the IFN-gamma production by NK cells after 16h activation in supernatant of DC matured in different clinical cocktails.
Figure 3: graph showing the synergistic effect on IL-12 production upon exposure of dendritic cells to IFN gamma, FMKp and CL075.
Figure 4: graph showing the synergistic effect on IL-12 production upon exposure of dendritic cells to of IFN gamma, FMKp and CL075 in a single donor.
Figure 5: graph showing the synergistic effect of exposure of dendritic cells to of IFN gamma, FMKp and CL075 in an NK activation assay.
Figure 6: Graph showing the production of IFN gamma by natural killer cells in relation to different growth factors.
Figure 7: Graph showing the production of IFN gamma in a Th 1 cell polarization assay in relation to different growth factors.
Figure 8: Graph showing an individual experiment that demonstrates the synergy on IL-12 production by maturing dendritic cells with IFNgamma, FMKp and poly IC.
Figure 9: Graph showing the interferon-gamma production by the T helper cells in a Th1 polarization assay in relation to Interferon gamma, FMKp and Poly IC.
Figure 10: Graph showing a summary of eight experiments on the activation of NK cells in supernatant of differently matured DC as measured by IFN-gamma production.

### Detailed description of the invention

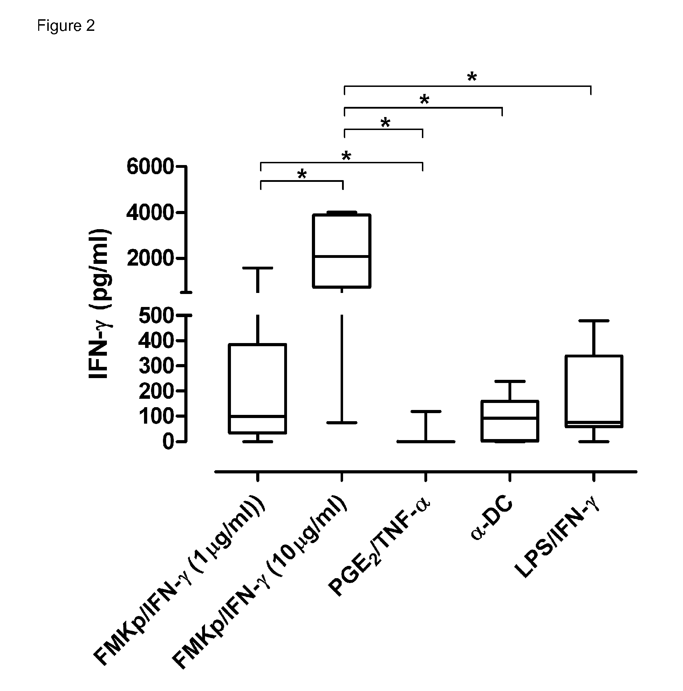

At first, we compared several maturation cocktails. We used FMKp/IFN-gamma matured DC as a reference and PGE2, TNF-alpha / ± IL6 and IL1beta matured DC (PGE2 DC) as controls. These latter DC are used thus far most studies and clinical trials. Immature dendritic cells (iDC) were cultured in the presence of FMKp and Interferon gamma (FI). The thus obtained mature DC were termed FI-DC. Characteristics of DC were analysed at 3 different levels: Thelper cell proliferation and polarization, IL12 production by DC and activation of Natural Killer cells. We found that a maturation cocktail consisting of 10 µg/ml FMKp and 500U/ml IFN gamma in addition to 50OU/ml IL-4 and 400U/ml GM-CSF performed best (figure 2).

We subsequently analysed if we could obtain DC that could induce even higher amounts of IL-12 and/or improved Th1 polarization and/or improved Natural Killer (NK) cell activation.

We found that certain combinations of maturation agents provided an unexpected synergistic effect. At first we were able to show that the combination of FMKp, IFN-gamma (FI) and CL075 provided unexpected advantages for all three parameters used herein.

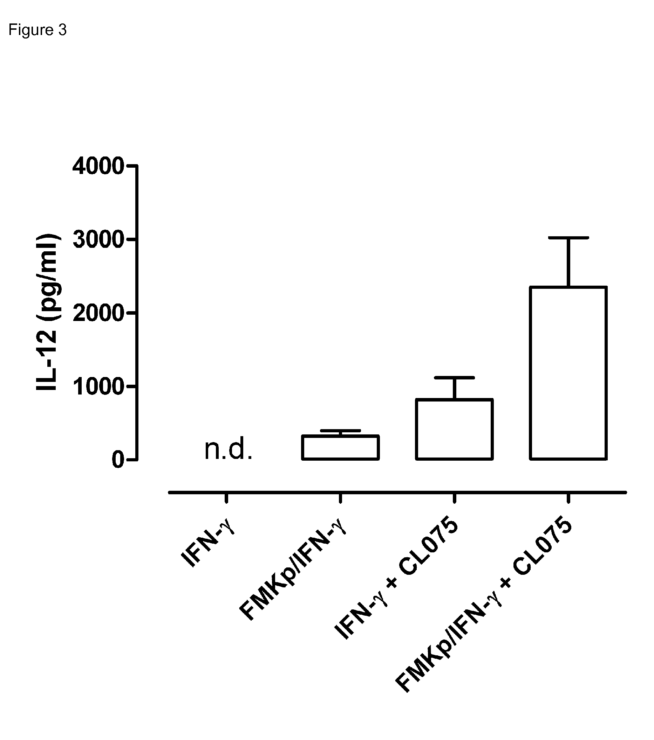

Figure 3 shows the synergistic effect on IL-12 production of FI plus CL075. The figure shows the average results of 5 experiments with a fixed dose of FMKp (dose = 1 microgram/ml) for FI DC and a fixed dose of CL075 (dose = 1 microgram/ml). It was observed that the IL12 production of FI + CL075 induced DC was higher than the sum of the IL-12 production of DC matured in any of the single constituents of the cocktail.

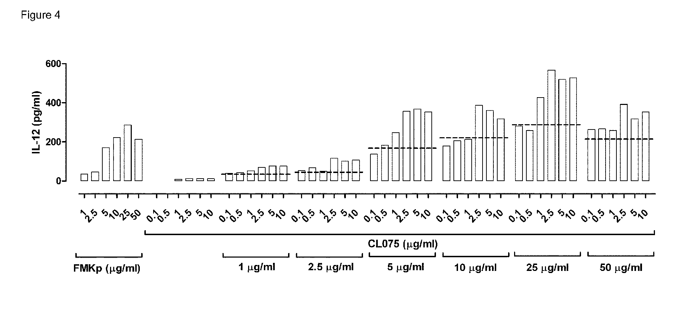

Figure 4 shows the data obtained with a single donor. Dose of FMKp is variable as well as the dose of CL075. The maximum response to be expected due to a complete additive effect of the various maturation agents is indicated with a horizontal line in figure 4. It is clear that most combinations of FI and CL075 showed a synergistic effect, i.e. more than the sum of the constituent parts.

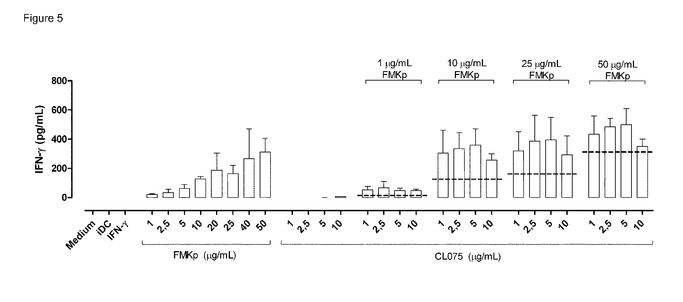

This cocktail also provided synergistic results in our NK activation assay (figure 5). It is clear from these data that NK activation by FMKp/IFN-gamma + CL075 DC supernatant is superior to activation with supernatant of DC matured with either FMKp/IFN-gamma or CL075 alone. The combination of the three compounds triggers a synergistic effect on NK cell activation. Figure 5 shows a representative experiment showing the amount NK cells-derived IFN-gamma after overnight stimulation with supernatant of DC matured with various doses of FMKp and/or various doses of CL075.

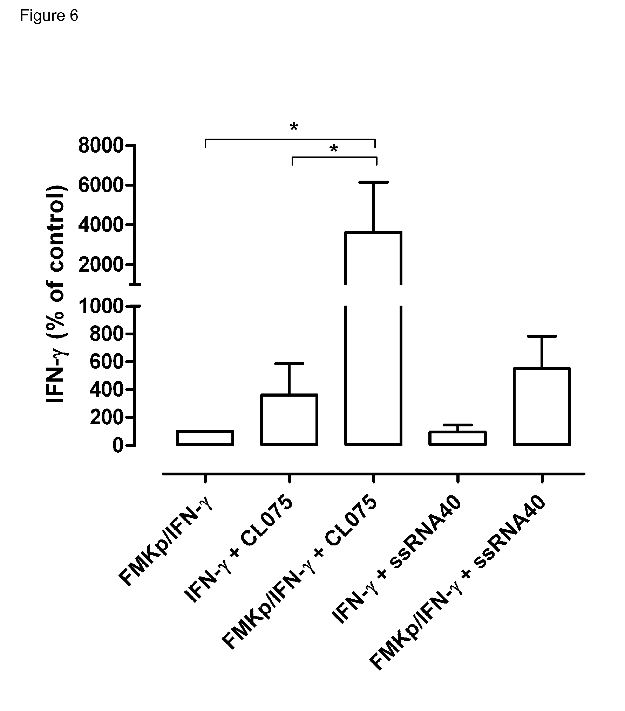

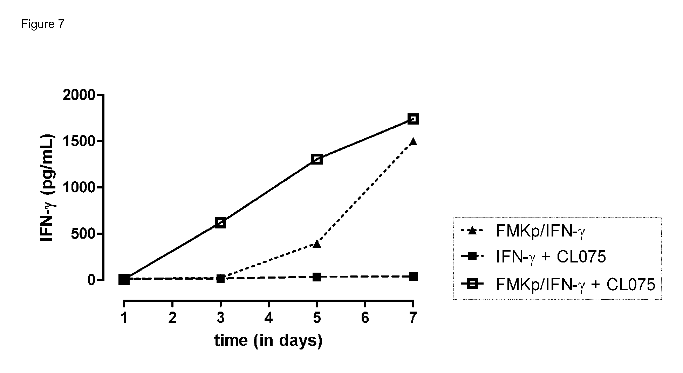

Figure 6 shows the sum of 7 experiments indicating the amount of Interferon-gamma produced by NK cells and demonstrates that FI + CL075 is superior in activating NK cells. FI alone induces activation of NK cells but far less than the combination. The dose of FMKp used was 1 microgram/ml; dose of CL075 used was 1 microgram/ml. These data also demonstrate that not all Toll-like receptor triggers do have a synergistic effect and that the response is specific for CL075 (in this case, ssRNA40 is used as a trigger for the identical TLR as CL075). The combination cocktail FI plus CL075 was also superior in our Th1 cell polarisation assay, as is shown in figure 7.

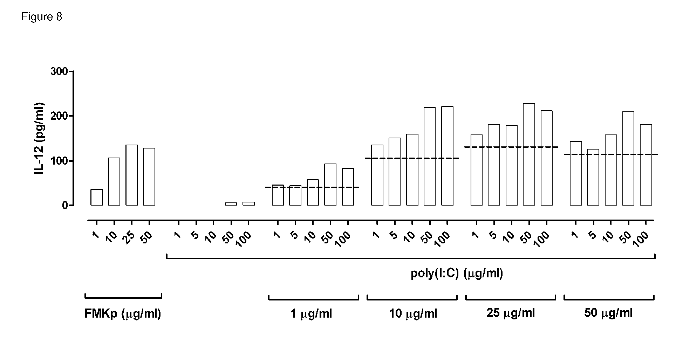

We also found that the combination of Poly IC and FMKp/IFN-gamma provided synergistic advantages. Figure 8 shows an individual experiment that demonstrates the synergy on IL-12 production by maturing dendritic cells with FI plus poly IC. Various doses of FMKp are used for the FI DC and various doses of poly IC. The IL-12 production obtained with the combination of FI plus poly IC is more than the sum of the constituent parts, showing true synergy.

It was observed that the amount of IL-12 produced by DC is variable between donors but that synergy is present in both high and low producers of IL-12. This can be of extreme clinical relevance since also in patients with low IL-12 producing DC, the production can be enhanced by the method of the invention.

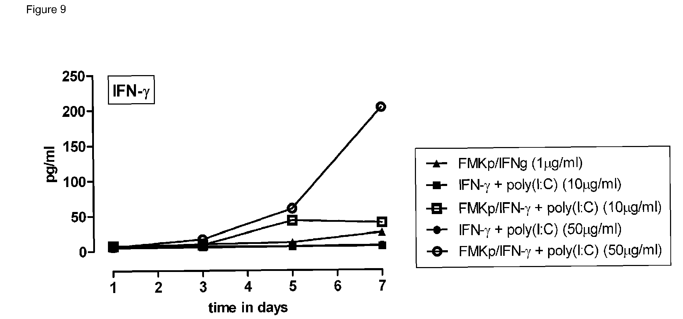

The combination of FI plus Poly IC also showed synergistic results in our Th1 polarization assay, as represented by interferon-gamma production by the T helper cells (figure 9). Only the combination of factors demonstrate relevant amounts of IFN gamma, illustrating the polarization of naïve T helper cells into Th1 cells, a crucial step in the activation of the immune system.

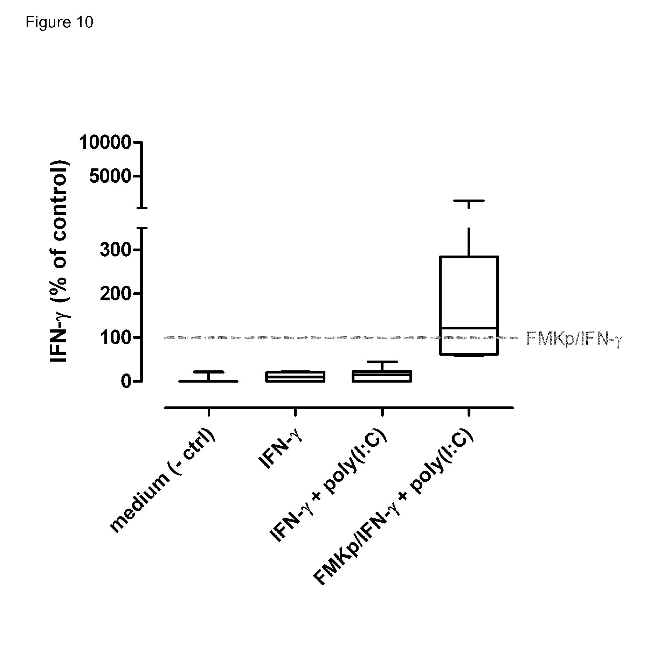

Figure 10 shows a summary of eight experiments on the activation of NK cells in supernatant of differently matured DC as measured by IFN-gamma production. The dose of FMKp used here is 1 microgram/ml. Poly IC was used in combination with IFN-gamma. Comparing the second group with the forth group and the dotted line (activation in FI-DC supernatant) show that the presence of the combination of three factors (FMKp, IFN-gamma and Poly IC) is superior to any other two factors either alone or in combination.

Accordingly, the invention relates to an ex vivo method for the production of mature dendritic cells wherein immature dendritic cells are exposed to a composition comprising FMKp and interferon gamma in combination with CL075 and/or Poly IC in a culture medium for a sufficient amount of time and wherein mature dendritic cells are isolated from the culture medium.

The maturation cocktail as described herein may also be used as a pharmaceutically active preparation in the treatment of humans or animals. Hence, the invention also relates to a composition comprising FMKp and interferon gamma in combination with CL075 and/or Poly IC for use in the induction of an immune response in an animal or human.

The invention may also be used to obtain superior dendritic cells. Hence, the invention also relates to mature dendritic cells obtainable by the method according to the invention.

Accordingly, the invention also relates to a composition comprising mature dendritic cells obtainable by the method according to the invention for use in treating cancer and/or infectious diseases. In an alternative wording, the invention relates to a method for the treatment of cancer and/or infectious diseases wherein a composition comprising dendritic cells obtainable by the method according to the invention is administered to a subject in need of such a treatment. In the alternative, the invention relates to a method for the treatment of cancer and/or infectious diseases wherein a composition comprising a combination of IFN gamma, FMKp and CL075 and/or poly (I:C) is administered to a subject in need of such a treatment.

### Example 1: Generation of Mature DC

Monocyte enrichment from leukapheresis products of five healthy volunteers (approved by Medical Ethics Committee of azM/ Maastricht University) was achieved by counterflow centrifugal elutriation using the Elutra® Cell Separation System monocyte enrichment application (Elutra®, Gambro BCT Europe, Zaventem, Belgium). Purity of enriched monocyte fraction exceeded 90% as assessed by flow cytometry. Monocytes were frozen at 50*10^6 cells per vial. Recovery after thawing generally exceeded 50-70%.

After thawing, monocytes were differentiated for 7 days in serum-free AIM-V medium (Gibco Life Technologies, Gaithesburg, MD, USA) at 2*10^6 cells/ml supplemented with IL-4 (2000U/mL; Strathmann Biotech AG, Hannover, Germany) and GM-CSF (400U/mL; Berlex, Richmond, CA, USA). After 7 days, immature DC (iDC) were harvested and cultured for two additional days in 24 well plates (Costar, New York, NY, USA) at 0.5 x 10^6 cells per well in maturation cocktails.

Maturation cocktails used herein contained IL-4 (500U/ml), GM-CSF (400U/ml) and IFN-gamma (500U/ml). They were supplemented with one or more of the following agents: FMKp (ranging form 1-50 microgram/ml), PRR-ligands (CL075 (0.1-10 microgram/ml), poly IC lyovec (0.1-5 microgram/ml) or poly IC LMW (0.1-100 microgram/ml). A reference maturation cocktail contained PGE2, TNF-α / IL-6 and IL-1beta.

On day 9, supernatants of mature DC (mDC) were harvested and IL-12 was determined by cytometric bead array (BD Biosciences, Erembodegem, Belgium). For NK activation washed supernatant was used. Therefore, the maturation cocktails were washed away 6h after the induction of maturation and cells were incubated for additional 42h in AIM-V medium.

### Example 2: Validation of assay systems, T helper cell proliferation.

We first set up a model system employing naïve CD4+ cells co-cultured with DC. The culture system of DC is described in example 1. In this experiment DC were matured with FMKp and IFn-gamma. Addition of different concentrations of recombinant IL-12 to this co-culture led to a dose-dependent increase of enlarged cells. We observed that the presence of FI-DC alone resulted in an increased number of cells as compared to the control wherein only T cells were cultured (without DC and additional IL-12), as shown in table 1. We also observed that the percentage of enlarged cells related to the total cell number of living cells increased (Table 1), indicating that addition of IL-12 to the co-culture of FI-DC with naive CD4+ T cells induces T cell expansion. These data illustrate that the amount of IL-12 is of great importance for the stimulation of T cells.

Table 1 T-cell expansion upon exposure to mature FMKp/IFN-gamma dendritic cells and different concentrations of recombinant IL12. On day five, cells were harvested and analyzed by flow cytometry.

| **Condition** | **Number of enlarged cells/ total number of cells x 100 (%)** |
|---|---|
| T cells (neg. control) | 2.3 |
| T cells + 20.000 DC | 18.5 |
| T cells + 20.000 DC+10 pg/ml IL12 | 14.6 |
| T cells + 20.000 DC +100 pg/ml IL12 | 21.5 |
| T cells + 20.000 DC+1000 pg/ml IL12 | 30.0 |
| T cells + 20.000 DC +10.000 pg/ml IL12 | 36.4 |

We concluded that T cell expansion is dependent on IL-12 concentration in a range between 10 and 1000 pg/ml IL-12.

### Example 3: Validation of assay systems, Polarisation of T helper1 cells.

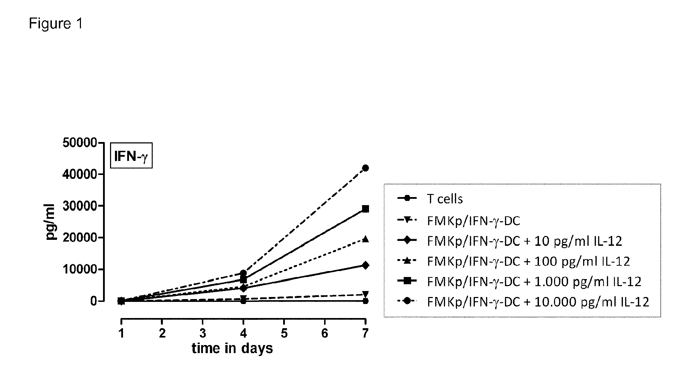

We also validated our experimental set-up for determining Th1 polarization. For that purpose, washed, 24h matured FMKp/IFN-gamma (FI) DC (See example 2) were co-cultured with naive (CD4+CD45RA+CD45RO-) allogeneic T cells during seven days, and TH specific cytokines were quantified in culture supernatant on day 1, 4 and 7 by CBA. The value of IFN-gamma is presented in figure 1. Again, various amounts of recombinant IL-12 are added to the co-culture. This figure demonstrates that mature FI-DC by themselves do not induce T helper polarization, but that additional IL-12 is needed. This exogenous supplementation studies proved that the requirement of IL-12 for Th1 polarization is quantitative instead of qualitative, as evidenced by a dose-dependent secretion of the Th1 cytokine IFN-gamma.

Naive CD4+ T cells were isolated from peripheral blood by negative immunomagnetic cell separation using a human naive CD4+ T cell isolation kit (Miltenyi Biotec, Germany) according to manufacturer's instructions. Briefly, density gradient centrifugation with lymphoprep (Axis-Shield, Oslo, Norway) was used to isolate peripheral blood mononuclear cells (PBMC) from fresh whole blood. PBMC were further incubated with a cocktail of biotinylated antibodies binding to all non-CD4+ T cells and to memory T cells. In a second incubation step anti-biotin microbeads were added and the cell suspension was finally added onto a column placed in a magnetic field leading to depletion of magnetically labelled cells. Naive CD4+ T cells were collected in the flow-trough. To achieve a higher purity the flow-trough was incubated a second time with biotinylated antibodies and microbeads. Incubation times were twice as long as indicated in the manual. Isolated CD4+CD45RA+CD45RO- exceeded 98% purity.

The capacity of differently matured DC to polarize naive CD4+ cells was evaluated by co-culturing mDC together with naive CD4+ T cells for one week. On day -1 iDC were harvested, transferred to round bottom 96-well plate (20.000cells/well) and matured with different cocktails as described in example 1. After 24h, autologous naive CD4+ T cells were added (50.000 cells/well) together with 50 microgram/mL pan-HLA-DR binding peptide (PADRE; AGVAAWTLKAAA, SEQ ID NO: 1), which functions as a superantigen. Throughout a week, each day supernatant was harvested and frozen at - 20°C for cytokine measurements by cytometric bead array (CBA; BD Biosciences). In addition, cells were harvested and lysed in RLT-buffer (containing beta-Mercaptoethanol) and frozen at -80°C for RNA isolation.

### Example 4: Validation of assay systems, Activation of natural Killer (NK) cells

NK cells were purified from buffy coats (obtained from Centre de transfusion sanguine, Liège, Belgium) by negative immunomagnetic cell separation using a human NK cell isolation kit (Miltenyi Biotec, Germany) according to the manufacturer's instructions. A minor modification was made concerning incubation times, which were elongated to 30min. Isolated NK cells were >95% CD56+CD3-.

Purified NK cells were resuspended in serum-free medium and placed at a density of 10*10^5 cells/well in a 96-well plate. 50 microliter of 48h cell-free culture supernatant (washed after 6h) from differently matured DC was added. As a negative control cells were incubated in AIM-V medium alone. All conditions were tested in triplicates. After 16h incubation at 37°C / 5% CO2, NK activation was determined by measuring total IFN-gamma production in the supernatant with ELISA.

Figure 2 shows the IFN-gamma production by NK cells after 16h activation insupernatant of DC matured in different clinical cocktails. IFN-gamma production is a marker of activation and was determined by ELISA. Wilcoxon-matched pairs test: FI-DC (1 microgram/mL FMKp) vs. clinical cocktails and FI-DC (10 microgram/ml FMKp) v clinical cocktails *, p<0.05. n=8 (except PGE2/TNF-α and FI 10 microgram/ml: n=6; LPSi/IFN-gamma: n=7). These data demonstrate that FI DC is the most optimal DC to activate NK cells and that the phenomenon is again dose (FMKp-) dependent. Higher doses (above 10 microgram/ml) did not induce stronger NK activation (data not shown).

SEQUENCE LISTING

<110>  Universiteit Maastricht and Academisch Ziekenhuis
       Maastricht

<120>  Method for the in vitro maturation of dendritic cells

<130>  185 EPP0

<160>  1

<170>  PatentIn version 3.5

<210>  1
<211>  12
<212>  PRT
<213>  homo sapiens

<400>  1

Ala Gly Val Ala Ala Trp Thr Leu Lys Ala Ala Ala
1               5                   10

### References

1. WHO: http://www.who.int/mediacentre/factsheets/fs297/en/ Accessed April 23, 2012.
2. Yang L, Carbone DP. Tumor-host immune interactions and dendritic cell dysfunction. Advances in Cancer Research. 2004 Jan; 9213-27.
3. Davis ID. Rational approaches to human cancer immunotherapy. Journal of Leukocyte Biology. 2003 Jan 1;73(1):3-29.
4. Dougan M, Dranoff G. Immune therapy for cancer. [Internet]. Annual review of Immunology. 2009 Jan 20;2783-117.
5. Ridgway D. The First 1000 Dendritic Cell Vaccinees. Cancer Invest. 2003;21 (6):873-86.
6. Colonna M, Trinchieri G, Liu Y-J. Plasmacytoid dendritic cells in immunity. Nature Immunology. 2004 Dec 17;5(12):1219-26.
7. Schreibelt G, Tel J, Sliepen KHEWJ, Benitez-Ribas D, Figdor CG, Adema GJ, et al. Toll-like receptor expression and function in human dendritic cell subsets: implications for dendritic cell-based anti-cancer immunotherapy. Cancer Immunology, Immunotherapy. 2010 ;59(10):1573-1582.
8. MacDonald KPA, Munster DJ, Clark GJ, Dzionek A, Schmitz J, Hart DNJ. Characterization of human blood dendritic cell subsets. Blood. 2002 Dec 15;100(13):4512-20.
9. Piccioli D, Tavarini S, Borgogni E, Steri V, Nuti S, Sammicheli C, et al. Functional specialization of human circulating CD16 and CD1c myeloid dendritic-cell subsets. Blood. 2007 Jun 15;109(12):5371-9.
10. Banchereau J, Palucka AK. Dendritic cells as therapeutic vaccines against cancer. Nature Reviews Immunology. 2005 Apr 1;5(4):296-306.
11. Banchereau J, Briere F, Caux C, Davoust J, Lebecque S, Liu YJ, et al. Immunobiology of dendritic cells. Annual Review of Immunology. 2000 Jan 28;18767-811.
12. Figdor CG, Vries IJM de, Lesterhuis WJ, Melief CJM. Dendritic cell immunotherapy: mapping the way. Nature Medicine. 2004 May 30;10(5):475-80.
13. Kalinski P, Okada H. Polarized dendritic cells as cancer vaccines: directing effector-type T cells to tumors. [Internet]. Seminars in immunology. 2010 Jun ;22(3):173-82.
14. Mailliard RB, Alber SM, Shen H, Watkins SC, Kirkwood JM, Herberman RB, et al. IL-18-induced CD83+CCR7+ NK helper cells. Journal of Experimental Medicine. 2005 Oct 3;202(7):941-53.
15. Raulet DH, Vance RE. Self-tolerance of natural killer cells. Nature Reviews Immunology. 2006 Jul 1;6(7):520-31.
16. Degli-Esposti MA, Smyth MJ. Close encounters of different kinds: dendritic cells and NK cells take centre stage. Nature Reviews Immunology. 2005 Feb 1;5(2):112-24.
17. Vasievich EA, Huang L. The suppressive tumor microenvironment: a challenge in cancer immunotherapy. Molecular Pharmaceutics. 2011 Jun 6;8(3):635-41.
18. Cooper MA, Bush JE, Fehniger TA, VanDeusen JB, Waite RE, Liu Y, et al. In vivo evidence for a dependence on interleukin 15 for survival of natural killer cells. Blood. 2002 Nov 15;100(10):3633-8.
19. Ferlazzo G, Pack M, Thomas D, Paludan C, Schmid D, Strowig T, et al. Distinct roles of IL-12 and IL-15 in human natural killer cell activation by dendritic cells from secondary lymphoid organs. Proceedings of the National Academy of Sciences USA. 2004 Nov 23;101(47):16606-11.
20. Vitale M, Della Chiesa M, Carlomagno S, Pende D, Aricò M, Moretta L, et al. NK-dependent DC maturation is mediated by TNFalpha and IFNgamma released upon engagement of the NKp30 triggering receptor. Blood. 2005 Jul 15;106(2):566-71.
21. Van Elssen CHMJ, Vanderlocht J, Frings PWH, Senden-Gijsbers BLMG, Schnijderberg MCA, Gelder M van, et al. Klebsiella pneumoniae-triggered DC recruit human NK cells in a CCR5-dependent manner leading to increased CCL19-responsiveness and activation of NK cells. European Journal of Immunology. 2010 Nov ;40(11):3138-49.
22. Mailliard RB, Son Y-I, Redlinger R, Coates PT, Giermasz A, Morel PA, et al. Dendritic Cells Mediate NK Cell Help for Th1 and CTL Responses: Two-Signal Requirement for the Induction of NK Cell Helper Function. J. Immunol. 2003 ;171(5):2366-2373.
23. Martin-Fontecha A, Thomsen LL, Brett S, Gerard C, Lipp M, Lanzavecchia A, et al. Induced recruitment of NK cells to lymph nodes provides IFN-gamma for T(H)1 priming. Nature Immunology. 2004 Dec 7;5(12):1260-5.
24. Kalinski P, Urban J, Narang R, Berk E, Wieckowski E, Muthuswamy R. Dendritic cell-based therapeutic cancer vaccines: what we have and what we need. Future Oncology. 2009 Apr 1 ;5(3):379-90.
25. Karimi K, Boudreau JE, Fraser K, Liu H, Delanghe J, Gauldie J, et al. Enhanced antitumor immunity elicited by dendritic cell vaccines is a result of their ability to engage both CTL and IFN gamma-producing NK cells. Molecular Therapy:. 2008 Feb 4;16(2):411-8.
26. Fernandez NC, Lozier A, Flament C, Ricciardi-Castagnoli P, Bellet D, Suter M, et al. Dendritic cells directly trigger NK cell functions: cross-talk relevant in innate anti-tumor immune responses in vivo. Nature Medicine. 1999 Apr 1;5(4):405-11.
27. Ferlazzo G. Human Dendritic Cells Activate Resting Natural Killer (NK) Cells and Are Recognized via the NKp30 Receptor by Activated NK Cells. Journal of Experimental Medicine. 2002 Feb 4;195(3):343-351.
28. Moretta A. Natural killer cells and dendritic cells: rendezvous in abused tissues. Nature Reviews Immunology. 2002 Dec 1;2(12):957-64.
29. Muzio M, Bosisio D, Polentarutti N, D□amico G, Stoppacciaro A, Mancinelli R, et al. Differential Expression and Regulation of Toll-Like Receptors (TLR) in Human Leukocytes: Selective Expression of TLR3 in Dendritic Cells. J. Immunol. 2000 ;164(11):5998-6004.
30. Padovan E, Landmann RM, De Libero G. How pattern recognition receptor triggering influences T cell responses: a new look into the system. Trends in Immunology. 2007 Jul ;28(7):308-14.
31. Lee MS, Kim Y-J. Signaling pathways downstream of pattern-recognition receptors and their cross talk. Annual review of biochemistry. 2007 Jan 19;76447-80.
32. Figdor CG, Kooyk Y van, Adema GJ. C-type lectin receptors on dendritic cells and Langerhans cells. Nature Reviews Immunology. 2002 Feb 1;2(2):77-84.
33. Fritz JH, Girardin SE. How Toll-like receptors and Nod-like receptors contribute to innate immunity in mammals. Journal of Endotoxin Research. 2005 Dec 1;11(6):390-394.
34. Cella M. Maturation, Activation, and Protection of Dendritic Cells Induced by Doublestranded RNA. Journal of Experimental Medicine. 1999 Mar 1;189(5):821-829.
35. Vanderlocht J, Elssen CHMJ van, Senden-Gijsbers BLMG, Meek B, Cloosen S, Libon C, et al. Increased tumor-specific CD8+ T cell induction by dendritic cells matured with a clinical grade TLR-agonist in combination with IFN-g. International Journal of Immunopathology and Pharmacology. 2010 ;23(1):35-50.
36. Oth T. Role of selective pattern recognition receptor-triggering in dendritic cell-mediated type 1 response. Internal Medicine, Haematology. 2010; Master Thesis 56.
37. Wang D, Dubois RN. Eicosanoids and cancer. Nature Reviews Cancer. 2010 Mar 19;10(3):181-93.
38. Boniface K, Bak-Jensen KS, Li Y, Blumenschein WM, McGeachy MJ, McClanahan TK, et al. Prostaglandin E2 regulates Th17 cell differentiation and function through cyclic AMP and EP2/EP4 receptor signaling. The Journal of Experimental Medicine. 2009 Mar 16;206(3):535-48.
39. Snijdewint FG, Kaliński P, Wierenga EA, Bos JD, Kapsenberg ML. Prostaglandin E2 differentially modulates cytokine secretion profiles of human T helper lymphocytes. Journal of Immunology. 1993 Jun 15;150(12):5321-9.
40. Zeddou M, Greimers R, Valensart N de, Nayjib B, Tasken K, Boniver J, et al. Prostaglandin E2 induces the expression of functional inhibitory CD94/NKG2A receptors in human CD8+ T lymphocytes by a cAMP-dependent protein kinase A type I pathway. Biochemical Pharmacology. 2005 Sep 1;70(5):714-24.
41. Ahmadi M, Emery DC, Morgan DJ. Prevention of both direct and cross-priming of antitumor CD8+ T-cell responses following overproduction of prostaglandin E2 by tumor cells in vivo. Cancer Research. 2008 Sep 15;68(18):7520-9.
42. Walker W, Rotondo D. Prostaglandin E2 is a potent regulator of interleukin-12- and interleukin-18-induced natural killer cell interferon-gamma synthesis. Immunology. 2004 Mar ;111(3):298-305.
43. Yakar I, Melamed R, Shakhar G, Shakhar K, Rosenne E, Abudarham N, et al. Prostaglandin e(2) suppresses NK activity in vivo and promotes postoperative tumor metastasis in rats. Annals of Surgical Oncology. 2003 May ;10(4):469-79.
44. Verdijk P, Aarntzen EHJG, Lesterhuis WJ, Boullart ACI, Kok E, Rossum MM van, et al. Limited amounts of dendritic cells migrate into the T-cell area of lymph nodes but have high immune activating potential in melanoma patients. Clinical Cancer Research. 2009 Apr 1;15(7):2531-40.
45. Kalinski P, Schuitemaker JHN, Hilkens CMU, Wierenga EA, Kapsenberg ML. Final Maturation of Dendritic Cells Is Associated with Impaired Responsiveness to IFN-{gamma} and to Bacterial IL-12 Inducers: Decreased Ability of Mature Dendritic Cells to Produce IL-12 During the Interaction with Th Cells. J. Immunol. 1999 ;162(6):3231-3236.
46. Cools N, Van Tendeloo VFI, Smits ELJM, Lenjou M, Nijs G, Van Bockstaele DR, et al. Immunosuppression induced by immature dendritic cells is mediated by TGF-beta/IL-10 double-positive CD4+ regulatory T cells. Journal of cellular and molecular medicine. 2008 Apr ;12(2):690-700.
47. Spranger S, Javorovic M, Bürdek M, Wilde S, Mosetter B, Tippmer S, et al. Generation of Th1-polarizing dendritic cells using the TLR7/8 agonist CL075. Journal of immunology (Baltimore, Md. : 1950). 2010 Jul 1;185(1):738-47.
48. Boullart ACI, Aarntzen EHJG, Verdijk P, Jacobs JFM, Schuurhuis DH, Benitez-Ribas D, et al. Maturation of monocyte-derived dendritic cells with Toll-like receptor 3 and 7/8 ligands combined with prostaglandin E2 results in high interleukin-12 production and cell migration. Cancer immunology, immunotherapy : CII. 2008 Nov ;57(11):1589-97.
49. Nguyen KB, Salazar-Mather TP, Dalod MY, Van Deusen JB, Wei X-qing, Liew FY, et al. Coordinated and Distinct Roles for IFN-{alpha]{beta}, IL-12, and IL-15 Regulation of NK Cell Responses to Viral Infection. J. Immunol. 2002 Oct 15;169(8):4279-4287.
50. Mailliard RB, Wankowicz-Kalinska A, Cai Q, Wesa A, Hilkens CM, Kapsenberg ML, et al. alpha-type-1 polarized dendritic cells: a novel immunization tool with optimized CTL-inducing activity. Cancer research. 2004 Sep 1;64(17):5934-7.

SEQUENCE LISTING

<110>  Universiteit Maastricht and Academisch Ziekenhuis
       Maastricht

<120>  Method for the in vitro maturation of dendritic cells

<130>  185 EPP0

<160>  1

<170>  PatentIn version 3.5

<210>  1
<211>  12
<212>  PRT
<213>  homo sapiens

<400>  1

Ala Gly Val Ala Ala Trp Thr Leu Lys Ala Ala Ala
1               5                   10

## Claims

1. Ex vivo method for the production of mature dendritic cells wherein immature dendritic cells are exposed to a composition comprising FMKp and interferon gamma in combination with CL075 and/or Poly IC in a culture medium for a sufficient amount of time and wherein mature dendritic cells are isolated from the culture medium.

2. Composition comprising FMKp and interferon gamma in combination with CL075 and/or Poly IC for use in the induction of an immune response in an animal or human.

3. Mature dendritic cells obtainable by the method according to claim 1.

4. Composition comprising mature dendritic cells according to claim 3 for use in treating cancer and/or infectious diseases.
